# EUROPÄISCHE PATENTSCHRIFT

(11) **EP 1 778 114 B1**
(45) Veröffentlichungstag und Bekanntmachung des Hinweises auf die Patenterteilung: **18.01.2012**
(21) Anmeldenummer: 05774846.9
(22) Anmeldetag: 15.07.2005
(51) Int. Cl.: A61B 18/14

(54) **ELEKTROCHIRURGISCHES INSTRUMENT**
ELECTROSURGICAL INSTRUMENT
INSTRUMENT ELECTROCHIRURGICAL

(30) Priorität: 11.08.2004 DE 102004039052; 18.11.2004 DE 102004055671
(43) Veröffentlichungstag der Anmeldung: 02.05.2007
(73) Patentinhaber: ERBE Elektromedizin GmbH, 72072 Tübingen (DE)
(72) Erfinder: HAFNER, Dieter, 72072 Tübingen (DE)
(74) Vertreter: Bohnenberger, Johannes
(86) Internationale Anmeldenummer: PCT/EP2005/007738
(87) Internationale Veröffentlichungsnummer: WO 2006/018087

(56) Entgegenhaltungen:
- US-A- 5 458 598
- US-A1- 2001 037 109
- US-A1- 2004 049 185
- US-B1- 6 267 761

## Beschreibung

Die Erfindung betrifft ein elektrochirurgisches Instrument nach dem Oberbegriff des Patentanspruches 1.

Ein solches geht aus der US 2004/0049185 A1 hervor.

Elektrochirurgische Instrumente werden seit vielen Jahren in der Hochfrequenz-Chirurgie eingesetzt, um biologisches Gewebe insbesondere zu koagulieren, aber auch zu schneiden. Bei einer Koagulation wird ein hochfrequenter Strom durch das zu behandelnde Gewebe geleitet, so dass sich dieses aufgrund Eiweißkoagulation und Dehydratation verändert. Das Gewebe zieht sich dabei derart zusammen, dass die Gefäße verschlossen und Blutungen gestillt werden. Nach erfolgter Koagulation ist das Gewebe beispielsweise mittels eines mechanisch arbeitenden Schneidinstruments durchtrennbar.

Elektrochirurgische Vorgänge sind sowohl monopolar als auch bipolar durchführbar. Bei der monopolaren Technik weist das elektrochirurgische Instrument nur eine einzige Stromzuführung auf, das zu behandelnde Gewebe (bzw. ein Patient) ist demgemäß auf das andere Potential zu legen. Immer mehr an Bedeutung gewinnen jedoch bipolare Instrumente, die mit zwei voneinander elektrisch isolierten Abschnitten ausgebildet sind. Der Stromweg zwischen den Elektrodenteilen ist damit kalkulierbar und verläuft nicht weite Strecken durch den Körper des Patienten. Damit reduziert sich die Beeinflussung von beispielsweise Herzschrittmachern oder sonstigen Geräten, die während der Operation an dem Patienten angeschlossen sind.

Bipolare Koagulationsinstrumente weisen im Wesentlichen zwei gelenkig miteinander verbundene Branchen auf, an deren proximalen Enden Griffeinrichtungen zur Handhabung der Branchen vorgesehen sind. An distalen Enden der Branchen befinden sich Elektrodenteile mit Koagulationsflächen zum Fassen von Gewebe und zum Durchleiten des Koagulationsstromes durch das Gewebe. Der von einem HF-Generator gelieferte HF-Strom wird dazu über Stromzuführungseinrichtungen zu den Elektrodenteilen des bipolaren Instruments geleitet.

Problematisch bei herkömmlichen elektrochirurgischen Instrumenten ist es jedoch, dass das einmal zwischen den Elektrodenteilen gefasste Gewebe leicht abgleitet bzw. abrutscht. Dies ist insbesondere dann mit Komplikationen verbunden, wenn das Operationsgebiet für den Chirurgen schlecht einsehbar ist, z. B. bei endoskopischen Eingriffen oder bei stark blutenden Gewebeabschnitten, und ein erneutes Aufgreifen des Gewebes Schwierigkeiten bereitet.

Insofern werden handelsübliche Instrumente häufig mit strukturierten Elektrodenteilen, d. h. mit strukturierten Koagulationsflächen ausgebildet, um das Gewebe sicher zwischen den Koagulationsflächen der Elektrodenteile zu arretieren. Die Elektrodenteile weisen dazu beispielsweise wellenförmige Koagulationsflächen auf.

Die Strukturierung der Koagulationsflächen ist jedoch mit vielen Nachteilen verbunden. Bereits die Herstellung derartiger Koagulationsflächen bedarf eines hohen Fertigungsaufwandes. Zudem fördert die Strukturierung ein Anhaften von Gewebe an den Flächen während des Eingriffs, so dass auch ein Wiederaufbereiten nur mit hohem Aufwand durchführbar ist. Die komplizierte Geometrie erschwert außerdem ein Nacharbeiten der Koagulationsflächen.

Der Erfindung liegt daher die Aufgabe zugrunde, ein elektrochirurgisches Instrument zum Koagulieren der eingangs genannten Art dahin gehend weiterzubilden, dass ein zu behandelndes Gefäß oder Gewebe sicher zwischen Elektrodenteilen arretierbar und dabei das Instrument, insbesondere Koagulationsflächen des Instruments, einfach herstell- und wiederaufbereitbar sind.

Diese Aufgabe wird durch ein elektrochirurgisches Instrument nach Patentanspruch 1 gelöst.

Insbesondere wird die Aufgabe durch ein elektrochirurgisches Instrument gelöst, das zwei gelenkig miteinander verbundene Branchen umfasst, die entsprechend einem Schneidoder Klemmwerkzeug betätigbar sind. Ferner umfasst das Instrument einander gegenüberliegende Elektrodenteile mit Koagulationsflächen an distalen Enden der Branchen zum Fassen von einem Gefäß oder Gewebe und zum Durchleiten eines Koagulationsstromes durch das Gefäß oder Gewebe zu dessen Koagulation, sowie Stromzuführungseinrichtungen zum Zuführen des Koagulationsstromes zu den Elektrodenteilen von einem HF-Generator. Eine der Koagulationsflächen ist mindestens in einem ersten mittleren Abschnitt konvex, die ihr gegenüberliegende Koagulationsfläche ist mindesten in einem zweiten mittleren Abschnitt konkav gekrümmt, wobei ein Krümmungsradius der konkav gekrümmten Koagulationsfläche mindestens in dem zweiten mittleren Abschnitt größer ist als ein Krümmungsradius der konvex gekrümmten Koagulationsfläche in dem ersten mittleren Abschnitt und wobei die Krümmungen um Längsachsen der distalen Enden derart verlaufen, dass das zwischen den distalen Enden gehaltene und senkrecht zu den Längsachsen verlaufende Gefäß oder Gewebe mit zu dem ersten und zweiten mittleren Abschnitt hin steigender Pressung gehalten wird.

Die Begriffe "konvex" und "konkav" sind in diesem Zusammenhang nicht nur als im Bogen gerundet zu verstehen. Vielmehr ist mit den Termini jede Art von Erhabenheit bzw. Einbuchtung zu verstehen, also z. B. auch eine dachförmige Erhabenheit und dementsprechend eine V-förmige Einbuchtung.

Ein wesentlicher Punkt der Erfindung liegt darin, dass durch die unterschiedlich gekrümmte Ausgestaltung der Koagulationsflächen eine Berührung der Koagulationsflächen mathematisch betrachtet nur an deren Scheitellinien erfolgen kann. Das heißt, zwischen den Koagulationsflächen ist ein Bereich maximaler Nähe ausgebildet, der sich symmetrisch um die Scheitellinien der Koagulationsflächen erstreckt. In diesem Bereich wird das Gewebe bei zusammengeführten Branchen aufgrund der erhöhten Pressung gegenüber übrigen Koagulationsbereichen besonders stark zusammengedrückt und damit sicher zwischen den Elektrodenteilen arretiert.

Vorteilhafterweise sind die oben beschriebenen Probleme bei herkömmlichen elektrochirurgischen Instrumenten mit der erfindungsgemäßen Vorrichtung beseitigt, da die einfache, glatte Geometrie einfach herstellbar ist, einem Anhaften von Gewebe während des Eingriffs entgegenwirkt und leicht wiederaufbereitet und nachbearbeitet werden kann. Zudem wird an den Bereichen hoher Pressung, also hohen Drucks, bedingt durch die hohe Klemmkraft ein sicheres Verschließen des Gefäßes oder Gewebes erreicht.

In einer ersten vorteilhaften Ausführungsform ist an mindestens einer der Koagulationsflächen im mittleren Abschnitt ein Isolationsabschnitt ausgebildet, so dass ein direkter elektrischer Kontakt zwischen den Koagulationsflächen vermeidbar ist. Aufgrund wärmeleitender Eigenschaften des Isolationsabschnittes wird auch an diesem eine Koagulation des Gewebes gewährleistet.

In einer weiteren bevorzugten Ausführungsform ist der Isolationsabschnitt derart an der mindestens einen Koagulationsfläche ausgebildet, dass er über diese hervorsteht. In diesem Falle dient der Isolationsabschnitt nicht nur der Isolation, sondern auch dazu, das zu behandelnde Gewebe mehrfach zu knicken und so eine verbesserte Arretierung des Gewebes zwischen den distalen Enden des elektrochirurgischen Instruments zu erreichen. Zudem wird die Pressung an dem Isolationsabschnitt weiter erhöht.

Vorzugsweise ist der Isolationsabschnitt aus mehreren Teilabschnitten ausgebildet. Dies ermöglicht eine besonders sichere Arretierung des Gewebes zwischen den Elektrodenteilen, weil das Gewebe mehrfach an Kanten der Teilabschnitte des Isolationsabschnittes geknickt wird.

In einer weiteren bevorzugten Ausführungsform ist der Isolationsabschnitt derart an der mindestens einen Koagulationsfläche ausgebildet, dass er sich durchgängig entlang einer Scheitellinie der Koagulationsfläche erstreckt und im Wesentlichen bündig mit dieser abschließt. Dies ist deshalb möglich, weil der Isolationsabschnitt an dem jeweiligen mittleren Abschnitt der entsprechenden Koagulationsfläche vorzusehen ist und so bei einem Zusammenführen der Branchen die gegenüberliegende Koagulationsfläche zuerst und ausschließlich erreicht. Vorteilhafterweise ist der Isolationsabschnitt bei dieser Ausführungsform geschützt in dem jeweiligen Elektrodenteil und somit sicher vor Verschleiß untergebracht.

Eine erfindungsgemäße Lösung sieht vor, den Isolationsabschnitt aus Keramik oder Diamant auszubilden. Vorteilhafterweise weisen Keramik und Diamant u. a. eine hohe Korrosionsbeständigkeit und eine hohe Verschleißfestigkeit gegenüber mechanischer Belastung auf.

Eine zwischen den Elektrodenteilen einen Kurzschluss verhindernde Einrichtung kann beispielsweise auch an den Branchen vorgesehen sein. Ist an diesen z. B. ein Abstandshalter angeordnet, können die Branchen nicht vollständig zusammengeführt werden, ein Abstand zwischen den Elektrodenteilen bleibt bestehen.

Vorteilhafterweise weist mindestens ein Elektrodenteil mindestens einen Durchlassbereich für ein Schneidinstrument zum Durchführen eines Schneidvorgangs auf, so dass mindestens ein Abschnitt des Durchlassbereichs als Führungsspalt für das Schneidinstrument vorgesehen und dieses an dem eingeklemmten Gewebe ansetzbar ist. Der Führungsspalt ermöglicht einen präzisen Schnitt an dem Gewebe, insbesondere bei mechanisch zu bedienenden Schneidinstrumenten.

Bei einer Ausgestaltung der Elektrodenteile mit gekrümmten Koagulationsflächen, wie oben beschrieben, bilden die Elektrodenteile Spannbereiche aus, so dass das Gewebe durch die Spannbereiche in Richtung deren Endbereiche gezogen, d. h. gestreckt wird. Das unter Spannung stehende Gewebe ist dann mittels des mechanisch arbeitenden Schneidinstruments leichter zu schneiden, weil sich Fasern des Gewebes quer zu einer Schneidrichtung ausrichten und das Gewebe dabei dünner wird. Damit ist eine an dem vorgespannten Gewebe zu dessen vollständiger Durchtrennung aufzubringende Kraft erheblich reduziert und einer mechanischen Belastung des Schneidinstruments, insbesondere einem Verschleiß von Schneidabschnitten wird entgegengewirkt. Auch ist der Schneidvorgang durch den Operateur leichter zu bewerkstelligen und das Instrument einfacher zu handhaben. Gleichzeitig ist das zu behandelnde Gewebe aufgrund der unterschiedlichen Krümmungsradien der Elektrodenteile bzw. der Spannbereiche insbesondere nahe einem Schneidbereich sicher zwischen den Spannbereichen arretiert.

Vorzugsweise teilt der Durchlassbereich die jeweiligen Elektrodenteile in mindestens zwei Bereiche, so dass die Elektrodenteile jeweils sich gegenüberliegende, parallel zueinander angeordnete Teilungsflächen aufweisen. Damit ist der Durchlassbereich über seinen gesamten Bereich als Führungsspalt nutzbar. Diese Art Führungsspalt ermöglicht einen äußerst präzisen Schnitt, weil das Schneidinstrument, insbesondere ein mechanisch zu bedienendes, besonders präzise führbar ist.

In einer weiteren bevorzugten Ausführungsform ist vorgesehen, dass der Durchlassbereich die jeweiligen Elektrodenteile in mindestens zwei Bereiche teilt, so dass die Elektrodenteile jeweils sich gegenüberliegende, sich in Richtung der Koagulationsflächen verjüngend zueinander angeordnete Teilungsflächen aufweisen. Da sich die Teilungsflächen der entsprechenden Elektrodenteile zu dem Schneidbereich an dem Gewebe hin annähern, ist dort weiterhin eine präzise Führung des Schneidinstruments gewährleistet. Der sich vom Schneidbereich weg gerichtete und aufgespreizte Teil des Durchlassbereichs eignet sich in besonderem Maße für eine Wiederaufbereitung, also Reinigung des Instruments nach erfolgtem Eingriff oder auch für das nachträgliche Aufbringen einer Beschichtung der Teilungsflächen mit einer beispielsweise verschleißresistenten Keramik, da durch die Ausgestaltung des Durchlassbereichs eine verbesserte Zugänglichkeit gewährleistet ist.

Vorzugsweise sind die Durchlassbereiche an den gegenüberliegenden Elektrodenteilen vorgesehen, wobei diese bei zusammengeführten Branchen im Wesentlichen fluchtend aneinander grenzen. Ist nur ein Durchlassbereich an einem Elektrodenteil ausgebildet, so eignet sich dieser insbesondere dafür, das Gewebe beispielsweise mittels eines chirurgischen Messers zu durchtrennen, wobei das Gewebe auf dem gegenüberliegenden Elektrodenteil vollständig, in gespanntem Zustand, aufliegt. Sind an beiden Elektrodenteilen Durchlassbereiche vorgesehen, so ist beispielsweise eine chirurgische Schere an dem koagulierten Gewebe ansetzbar und dieses auf einfache Weise zu durchtrennen. Zur Ausführung eines gut kalkulierbaren Schnittes sind die Durchlassbereiche vorzugsweise in den mittleren Abschnitten der Elektrodenteile angeordnet.

In einer bevorzugten Ausführungsform ist das Schneidinstrument mit dem elektrochirurgischen Instrument verbunden ausgebildet. Beispielsweise befindet sich das Schneidinstrument innerhalb einer der Branchen und kann bei Bedarf in eine Schneidposition gebracht werden. Damit ist ein Instrumentenwechsel vermeidbar, so dass ein Operationsverlauf nicht unterbrochen werden muss.
Bei in das Koagulationsinstrument integriertem Schneidinstrument sind vorzugsweise beide Elektrodenteile mit dem Durchlassbereich ausgebildet, damit das Schneidinstrument das Gewebe ungehindert erreichen kann.

Ist das Schneidinstrument nicht integrativ mit dem elektrochirurgischen Instrument ausgebildet, so ist der Führungsspalt derart auszulegen, dass ein von außen kommendes Schneidinstrument bei hinreichend genauer Führung an dem vorgespannten Gewebe ansetzbar ist.

Eine vorteilhafte Ausführungsform sieht vor, dass das Schneidinstrument mechanisch und/oder elektrisch betätigbar ist. So kann beispielsweise eine an einem Schaft ausgebildete Klinge an dem elektrochirurgischen Instrument vorgesehen sein, die während der Koagulation in der Branche untergebracht ist und für den Schneidvorgang an das Gewebe herangeführt wird. Das Positionieren der Klinge oder eines sonstigen Schneidinstruments und auch ein Vorschub können hierbei selbsttätig erfolgen oder auch von dem Chirurgen mechanisch durchgeführt werden.

Eine erfindungsgemäße Lösung sieht vor, dass das Schneidinstrument zum Schneiden mittels eines HF-Stromes ausgebildet und mit einer Steuerungseinheit verbunden ist, so dass der Schneidstrom in Abhängigkeit von Operationsphasen zuführbar ist. Der Operateur kann dann den Schneidvorgang derart steuern, dass dieser selbsttätig und optimiert abläuft.

Eine erfindungsgemäße Lösung sieht vor, dass an dem einen Elektrodenteil und/oder an dem gegenüberliegenden Elektrodenteil ein einen durch die Krümmungen bewirkten Spanneffekt an dem Gewebe unterstützendes Oberflächenprofil ausgebildet ist. Das Profil ist vorzugsweise an Endbereichen der jeweiligen Elektrodenteile ausgebildet und bewegt das Gewebe zusätzlich in einer durch die Elektrodenteile definierten Zugrichtung bzw. verhindert ein Zurückweichen des Gewebes entgegen dieser Zugrichtung.

Vorzugsweise ist das den Spanneffekt unterstützende Oberflächenprofil als Sägezahnprofil ausgebildet. Zähne des Profils können beispielsweise so angeordnet sein, dass sie während des Zusammenführens der Branchen immer weiter in das Gewebe greifen und dieses in Zugrichtung mitnehmen. Damit wird die Spannung im Gewebe deutlich erhöht. Es ist allerdings darauf zu achten, dass durch das Profil eine Verletzung des Gewebes vermieden wird, so dass die Zähne vorzugsweise als abgerundete Noppen ausgebildet sind.

Das Profil ist vorteilhafterweise derart ausgestaltet, dass das Gewebe bei einem leichten Öffnen der Branchen durch das Profil in seiner gespannten Position gehalten wird. Das Profil fungiert demgemäß als eine Anordnung von Widerhaken.

In einer weiteren bevorzugten Ausführungsform ist der oder jeder Isolationsabschnitt als das den Spanneffekt unterstützendes Oberflächenprofil ausgebildet. Damit wird auf einfachste Weise sowohl der Kurzschluss zwischen den Elektrodenteilen vermieden, als auch das Spannen des Gewebes verstärkt.

Elektrochirurgische Instrumente dieser Art können beispielsweise für den Einsatz am eröffneten Körper ausgebildet sein. Das Prinzip der gekrümmt ausgebildeten Elektrodenteile ist jedoch auch für in der Endoskopie eingesetzte Instrumente anwendbar. Die an den Branchen befestigten Elektrodenteile und ggf. das Schneidinstrument sind dann beispielsweise über einen an einem Schaft befestigten Handgriff zu betätigen oder aber es ist eine Steuerungseinheit vorgesehen, so dass eine Betätigung der Elektrodenteile und/oder des Schneidinstruments über diese gesteuert wird. So ist das elektrochirurgische Instrument vorzugsweise als laparoskopisches Instrument ausgebildet.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen
- Fig. 1 ein perspektivisch dargestelltes elektrochirurgisches Instrument mit einer erfindungsgemäßen Elektrodenanordnung in einer ersten bevorzugten Ausführungs form;
- Fig. 2 ein perspektivisch dargestelltes elektrochirurgisches Instrument mit einer erfindungsgemäßen Elektrodenanordnung in einer zweiten bevorzugten Ausführungs form;
- Fig. 3 die schematisch im Schnitt dargestellte Elektrodenanordnung in Vorderansicht gemäß der ersten bevorzugten Ausführungsform aus Fig. 1;
- Fig. 4 eine schematisch im Schnitt dargestellte Elektrodenanordnung in Vorderansicht gemäß einer dritten bevorzugten Ausführungsform;
- Fig. 5 eine schematisch im Schnitt dargestellte Elektrodenanordnung in Vorderansicht gemäß einer vierten bevorzugten Ausführungsform;
- Fig. 6 eine schematisch im Schnitt dargestellte Elektrodenanordnung in Vorderansicht gemäß einer fünften bevorzugten Ausführungsform.

In der nachfolgenden Beschreibung werden für gleiche und gleich wirkende Teile dieselben Bezugsziffern verwendet.

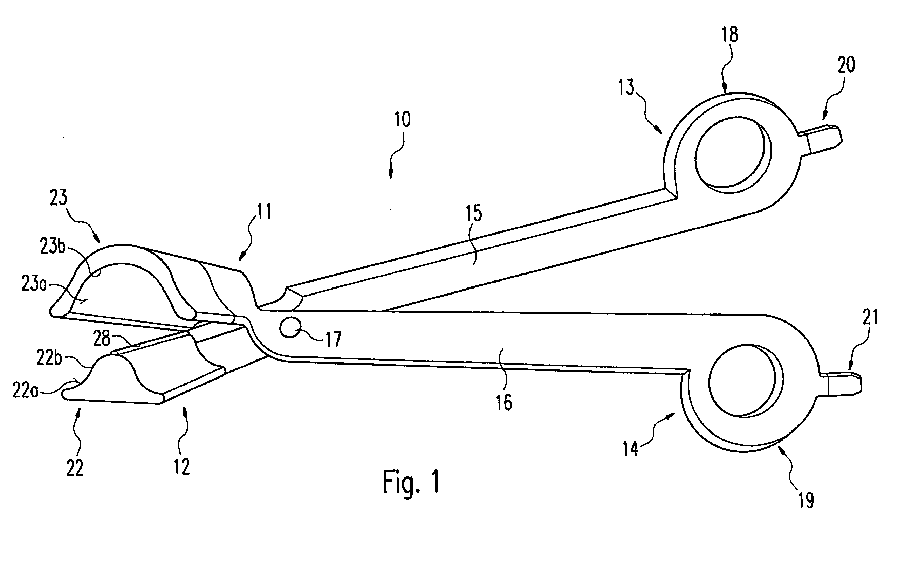

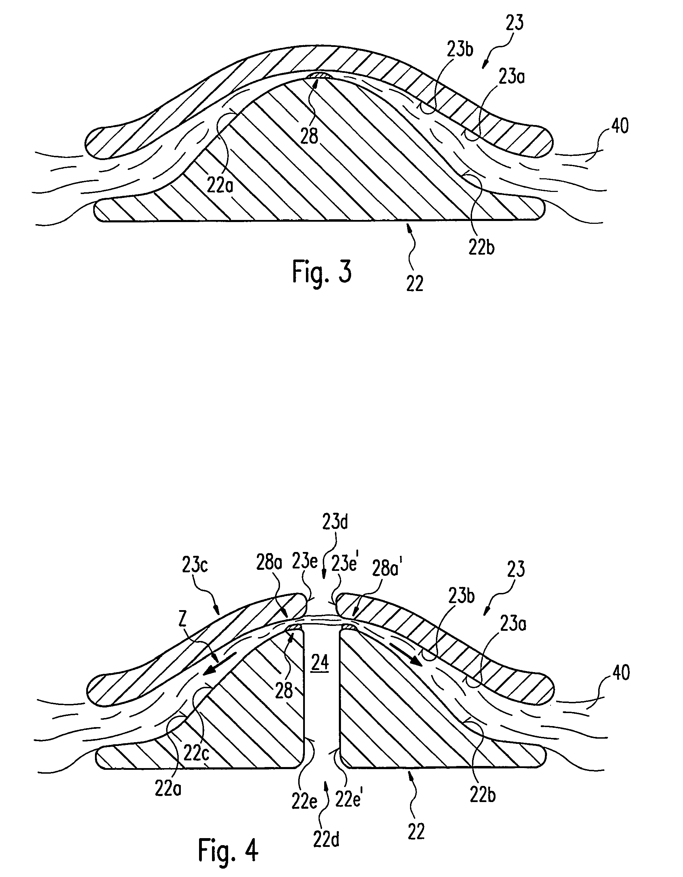

Fig. 1 zeigt ein perspektivisch dargestelltes elektrochirurgisches Instrument 10 mit einer erfindungsgemäßen Elektrodenanordnung in einer ersten bevorzugten Ausführungsform. Fig. 3 zeigt die Elektrodenanordnung der ersten bevorzugten Ausführungsform in einer Vorderansicht im Schnitt. Das Instrument 10 ist für einen Eingriff am eröffneten Körper ausgebildet. In der Abbildung sind mit den Bezugsziffern 15 und 16 zwei Branchen des elektrochirurgischen Instruments 10 bezeichnet. Die beiden Branchen 15, 16 sind über eine Achse 17 miteinander verbunden und um diese verschwenkbar. Sie weisen mit Elektrodenteilen 22, 23 versehene distale Enden 11, 12 auf, wobei sich die Elektrodenteile 22, 23 einander gegenüberliegen. Mit Hilfe der Elektrodenteile 22, 23, die Koagulationsflächen 22a, 23a aufweisen, lässt sich beispielsweise ein Gefäß oder Gewebe 40 fassen und durch Zuleitung von hochfrequentem Strom koagulieren. Ferner sind Griffteile 18, 19 vorgesehen, die an jeweilige proximale Enden 13, 14 der Branchen 15, 16 anschließen. Die proximalen Enden 13, 14 der Branchen 15, 16 enden jeweils in einem Stromanschlusselement bzw. einer Stromzuführungseinrichtung 20, 21 zum Anschließen des elektrochirurgischen Instruments 10 an einen (nicht dargestellten) HF-Generator, der eine HF-Spannung erzeugt, so dass der HF-Strom beispielsweise durch in dem Instrument 10 laufende elektrische Leitungen (nicht gezeigt) den Elektrodenteilen 22, 23 zugeführt werden kann.

Das elektrochirurgische Instrument 10 ist derart ausgebildet, dass sich ein Elektrodenteil 23 bei Züsammenführung der Branchen 15, 16 über das andere Elektrodenteil 22 stülpt, d. h., dieses abdeckt. Wie aus der Abbildung ersichtlich, sind die Elektrodenteile 22, 23 gekrümmt ausgebildet. Dabei weist ein Elektrodenteil 22 in einem ersten mittleren Abschnitt eine konvexe Krümmung 22b auf und das Elektrodenteil 23, das dem konvexen Elektrodenteil gegenüberliegt, weist in einem zweiten mittleren Abschnitt eine konkave Krümmung 23b auf. Ein Krümmungsradius der konkav gekrümmten Koagulationsfläche ist größer ist als ein Krümmungsradius der konvex gekrümmten Koagulationsfläche. Die Krümmungen 22b, 23b verlaufen um Längsachsen der distalen Enden 11, 12 derart, dass das zwischen den distalen Enden 11, 12 gehaltene und senkrecht zu den Längsachsen verlaufende Gefäß oder Gewebe 40 mit zu dem ersten und zweiten mittleren Abschnitt hin steigender Pressung gehalten wird.

Aufgrund der gekrümmten Ausgestaltung der Koagulationsflächen 22a, 23a bzw. der Elektrodenteile 22, 23 kann eine Berührung der Koagulationsflächen 22a, 23a mathematisch betrachtet nur an deren Scheitellinien erfolgen. Das heißt, zwischen den Koagulationsflächen 22a, 23a ist ein Bereich maximaler Nähe ausgebildet, der sich symmetrisch um die Scheitellinien der Koagulationsflächen 22a, 23a erstreckt. In diesem Bereich wird das Gewebe 40 bei zusammengeführten Branchen 15, 16 aufgrund der erhöhten Pressung gegenüber übrigen Koagulationsbereichen besonders stark zusammengedrückt und damit sicher zwischen den Elektrodenteilen 22, 23 arretiert.

Die einfache, glatte Geometrie der Koagulationsflächen 22a, 23a ist einfach und kostengünstig herstellbar. Auch wird einem Anhaften von Geweberesten während der Operation entgegengewirkt, so dass die Flächen bei einer Wiederaufbereitung leichter zu reinigen sind. Ein etwaiges Nachbearbeiten der Koagulationsflächen 22a, 23a lässt sich aufgrund der einfachen Geometrie leicht bewerkstelligen. Zudem wird an den Bereichen hoher Pressung, also hohen Drucks, bedingt durch die hohe Klemmkraft ein sicheres Verschließen des Gefäßes oder Gewebes 40 erreicht.

An der Scheitellinie des konvex gekrümmten Elektrodenteils 22 ist an der Koagulationsfläche 22a ein durchgehender Isolationsabschnitt 28 angeordnet. Der Isolationsabschnitt 28 verhindert zuverlässig eine vollständige Kontaktierung der Koagulationsflächen 22a, 23a und damit einen Kurzschluss. Zudem verstärkt der Isolationsabschnitt 28 die Pressung an dem Gewebe 40. Da das Gewebe 40 an dem Isolationsabschnitt 28 zusätzlich geknickt wird, kann hier eine noch weiter verbesserte Arretierung des Gewebes 40 erreicht werden.

Alternativ wäre es möglich, den Isolationsabschnitt derart an der Koagulationsfläche 22a auszubilden, dass er sich ebenfalls durchgängig entlang der Scheitellinie der Koagulationsfläche 22a erstreckt, im Wesentlichen aber bündig mit dieser abschließt. Der Isolationsabschnitt ist dann in die Koagulationsfläche 22a eingelegt. Dies ist deshalb möglich, weil der Isolationsabschnitt an dem ersten mittleren Abschnitt der Koagulationsfläche 22a vorgesehen wäre und so bei einem Zusammenführen der Branchen 15, 16 die gegenüberliegende Koagulationsfläche 23a zuerst und ausschließlich erreichen würde. Vorteilhafterweise wäre der Isolationsabschnitt bei dieser Ausführungsform geschützt in dem entsprechenden Elektrodenteil 22 und somit sicher vor Verschleiß untergebracht.

Vorzugsweise ist der Isolationsabschnitt 28 aus Keramik oder Diamant ausgebildet. Beide Materialien weisen u. a. eine hohe Korrosionsbeständigkeit und eine hohe Verschleißfestigkeit gegenüber mechanischer Belastung auf.

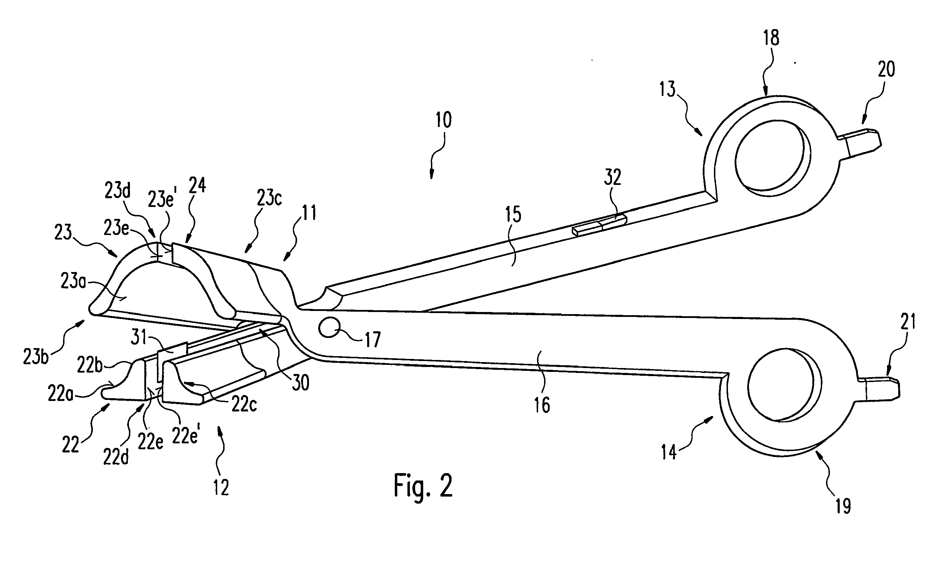

Fig. 2 zeigt ein perspektivisch dargestelltes elektrochirurgisches Instrument 10 mit einer erfindungsgemäßen Elektrodenanordnung in einer zweiten bevorzugten Ausführungsform. Das Instrument ist im Prinzip ähnlich dem in Fig. 1 gezeigten aufgebaut, weist aber geteilte Elektrodenteile 22, 23 auf.

Die Elektrodenteile 22, 23 weisen Durchlassbereiche 22d, 23d auf, die einen Führungsspalt 24 für ein Schneidinstrument 30 ausbilden. An einem eingeklemmten Gewebe ist demgemäß das Schneidinstrument 30 zum Durchführen eines Schneidvorgangs ansetzbar. Durch die gekrümmten Elektrodenteile 22, 23 wird das Gewebe in Richtung von Endbereichen der Elektrodenteile 22, 23 gezogen, d. h. in einer Zugrichtung gestreckt. Die Elektrodenteile 22, 23 bilden demgemäß Spannbereiche 22c, 23c aus. Damit lässt sich das Gewebe leichter schneiden, da sich Fasern des Gewebes quer zu einer Schneidrichtung ausrichten und das Gewebe dabei dünner wird. Bei einem Schließen von Branchen 15, 16 ist das Gewebe dann zwischen den Branchen 15, 16 in gespanntem Zustand fixiert. In diesem Ausführungsbeispiel sind die Elektrodenteile 22, 23 im Wesentlichen vollständig als Spannbereiche 22c, 23c ausgebildet. Alternativ ist es möglich, dass nur Abschnitte der Elektrodenteile 22, 23 Spannbereiche ausbilden.

Der Führungsspalt 24 ermöglicht einen präzisen Schnitt an dem Gewebe, weil das Schneidinstrument 30 entlang dem Führungsspalt 24 geführt wird. Dies ist insbesondere dann vorteilhaft, wenn das Schneidinstrument mechanisch bedient wird. Gleichzeitig verhindern die Spannbereiche 22c, 23c, dass das Gewebe in den Führungsspalt 24 eindringt, da das Gewebe aufgrund der Spannung von und aus diesem weggezogen wird.

Da beide Spannbereiche 22c, 23c Durchlassbereiche 22d, 23d aufweisen, sind diese fluchtend aneinander angeordnet. Nur so lässt sich eine präzise Führung des Schneidinstruments 30 gewährleisten.

Wie in diesem Ausführungsbeispiel gezeigt, teilen die Durchlassbereiche 22d, 23d die jeweiligen Elektrodenteile 22, 23 in mindestens zwei Bereiche, so dass die Elektrodenteile 22, 23 jeweils sich gegenüberliegende, parallel zueinander angeordnete Teilungsflächen 22e, 22e' bzw. 23e, 23e' aufweisen. Damit sind die Durchlassbereiche 22d, 23d über ihre gesamte Länge als Führungsspalt 24 nutzbar. Diese Art Führungsspalt 24 ermöglicht einen äußerst präzisen Schnitt, weil das Schneidinstrument 30 besonders präzise führbar ist, insbesondere, wenn es mechanisch bedient wird.

Alternativ wäre es möglich, nur einen Durchlassbereich an einem Elektrodenteil auszubilden, so dass das Gewebe beispielsweise mittels eines chirurgischen Messers durchtrennbar ist. Dabei liegt das Gewebe auf dem gegenüberliegenden Elektrodenteil vollständig, in gespanntem Zustand, auf.

Das Schneidinstrument 30 weist eine Klinge 31 an einem Schaft auf und ist während einer Koagulationsphase innerhalb der Branche 15 untergebracht. Für den Schneidvorgang lässt sich das Schneidinstrument 30 an dem bereits koagulierten Gewebe positionieren und zum Durchtrennen des Gewebes mit einer definierten Vorschubgeschwindigkeit bewegen. Dies geschieht in diesem Ausführungsbeispiel beispielsweise durch eine das Schneidinstrument 30 ansteuernde (nicht gezeigte) Steuerungseinheit, die durch einen Fingerschalter 32 aktivierbar ist. Da das Schneidinstrument 30 in dem elektrochirurgischen Instrument 10 integriert ausgebildet ist, ist ein Instrumentenwechsel und damit eine Unterbrechung eines Operationsverlaufs vermeidbar.

Alternativ ist es möglich, das Schneidinstrument mechanisch durch den Anwender zu betätigen. Der Chirurg kann dann die Klinge 31 bei Bedarf durch die Branche 15 an und durch das Gewebe schieben.

Ist an dem elektrochirurgischen Instrument keine Vorrichtung zum Schneiden des Gewebes vorgesehen, so ist der Führungsspalt 24 derart auszulegen, dass ein von außen kommendes Schneidinstrument, z. B. eine chirurgische Schere, bei hinreichend genauer Führung an dem vorgespannten Gewebe ansetzbar ist.

In der praktischen Anwendung ist an dem elektrochirurgischen Instrument 10 ein Abstandshalter (nicht gezeigt) oder dergleichen zwischen den Elektrodenteilen 22, 23 einen Abstand haltende Vorrichtung ausgebildet, damit ein unmittelbarer Kontakt der Koagulationsflächen 22a, 23a der Elektrodenteile 22, 23 und damit ein Kurzschluss vermeidbar ist. Der Abstandshalter kann beispielsweise an einer der Branchen 15, 16 ausgebildet sein.

Die in Fig. 1 und Fig.2 gezeigten elektrochirurgische Instrumente 10 sind, wie bereits oben erwähnt, für den Einsatz am eröffneten Körper ausgebildet. Das Prinzip der mit den Spannbereichen 22c, 23c und den unterschiedlichen Krümmungsradien ausgebildeten Elektrodenteilen 22, 23 ist ebenso für die Endoskopie anzuwenden. Die an den Branchen befestigten Elektrodenteile und ggf. das Schneidinstrument sind dann beispielsweise über einen an einem Schaft befestigten Handgriff zu betätigen oder aber es ist eine Steuerungseinheit vorgesehen, so dass eine Betätigung der Elektrodenteile und/oder des Schneidinstruments über diese gesteuert wird.

Fig. 4 bis 6 zeigen jeweils eine stark vergrößerte Vorderansicht einer Elektrodenanordnung im Schnitt in einer dritten, vierten und fünften Ausführungsform. Die Elektrodenteile 22, 23 entsprechen im Wesentlichen der Ausgestaltung der in Fig. 2 gezeigten. Auch weisen diese als Führungsspalt 24 für ein Schneidinstrument dienende Durchlassbereiche 22d, 23d wie in Fig. 2 beschrieben auf. Bei diesen Ausführungsformen ist jeweils, wie bereits bei Fig. 1 beschrieben, das Elektrodenteil 22 in einem ersten mittleren Abschnitt konvex gekrümmt, während das gegenüberliegende Elektrodenteil 23 in einem zweiten mittleren Abschnitt eine konkave Krümmung aufweist. Ein Krümmungsradius der konkav gekrümmten Koagulationsfläche ist größer ist als ein Krümmungsradius der konvex gekrümmten Koagulationsfläche. Die Krümmungen 22b, 23b verlaufen um Längsachsen der distalen Enden derart, dass ein zwischen den distalen Enden gehaltenes und senkrecht zu den Längsachsen verlaufendes Gefäß oder Gewebe 40 mit zu dem ersten und zweiten mittleren Abschnitt hin steigender Pressung gehalten wird. Aufgrund der Krümmungen 22b, 23b sind die Elektrodenteile 22, 23 bei diesen Ausführungsformen als Spannbereiche 22c, 23c ausgebildet. Das Gewebe 40 wird aufgrund der Spannbereiche 22c, 23c zu Endbereichen der Elektrodenteile 22, 23 hin in einer Zugrichtung Z gestreckt. Damit richten sich Fasern des Gewebes 40 quer zu einer Schneidrichtung aus, so dass das Gewebe 40 leichter geschnitten werden kann, während es gleichzeitig sicher in den unterschiedlich gekrümmten Elektrodenteilen 22, 23 fixiert ist.

Fig. 4 zeigt die Elektrodenanordnung gemäß der dritten bevorzugten Ausführungsform. Diese unterscheidet sich im Wesentlichen von der in Fig. 2 gezeigten Elektrodenanordnung nur dadurch, dass ein aus zwei Teilabschnitten 28a, 28a' ausgebildeter, hervorstehender Isolationsabschnitt 28 unmittelbar benachbart einem Durchlassbereich 22d an dem durch den Durchlassbereich 22d in zwei Bereiche aufgeteilten, konvex ausgebildeten Elektrodenteil 22 vorgesehen ist. Vorzugsweise verlaufen die Teilabschnitte 28a, 28a' des Isolationsabschnitts 28 parallel einer Scheitellinie des Elektrodenteils 22. Damit wird ein Kurzschluss zwischen den Elektrodenteilen 22, 23 bei einem Zusammenführen derselben verhindert. Die Teilabschnitte 28a, 28a' des Isolationsabschnittes 28 unterstützen einerseits die Spannwirkung des Spannbereichs 22 und ermöglichen andererseits eine Knickung des eingespannten Gewebes 40. Damit ist dessen zuverlässige Arretierung zwischen den Elektrodenteilen 22, 23 gewährleistet.

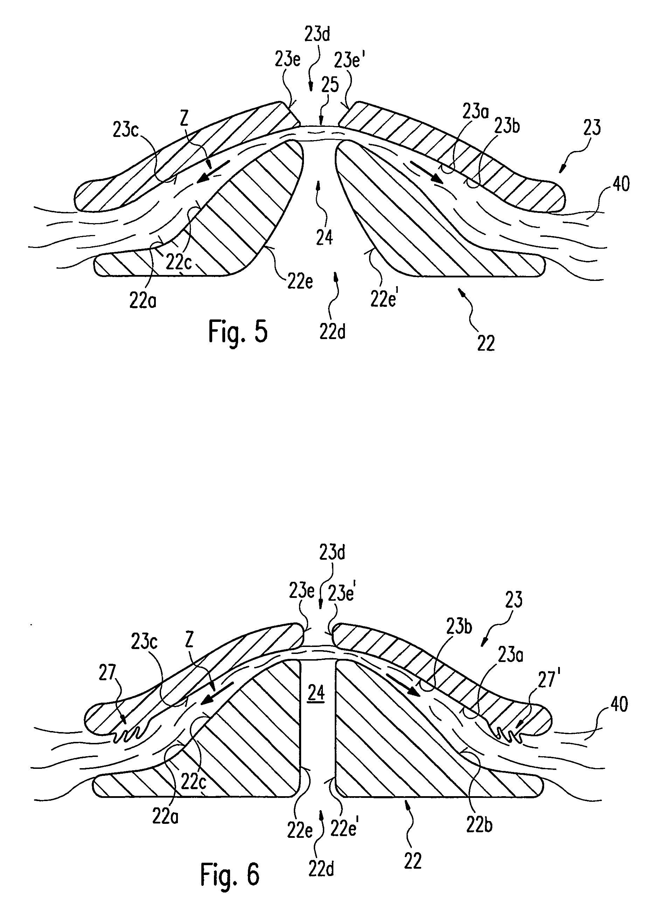

Fig. 5 zeigt ebenfalls eine Elektrodenanordnung gemäß einer vierten bevorzugten Ausführungsform. Auch hier sind die Elektrodenteile 22, 23 im Schnitt in Vorderansicht dargestellt. Die Elektrodenteile 22, 23 weisen Durchlassbereiche auf, die von den in Fig. 2 gezeigten verschieden sind. Die Durchlassbereiche 22d, 23d teilen die jeweiligen Elektrodenteile 22, 23 in zwei Bereiche derart, dass die Elektrodenteile 22, 23 jeweils sich gegenüberliegende, sich in Richtung zu Koagulationsflächen 22a, 23a hin verjüngend zueinander angeordnete Teilungsflächen 22e, 22e' bzw. 23e, 23e' aufweisen. Da sich die Teilungsflächen 22e, 22e', 23e, 23e' der entsprechenden Elektrodenteile 22, 23 zu einem Schneidbereich 25 hin annähern, ist dort weiterhin eine präzise Führung eines Schneidinstruments gewährleistet. Der sich vom Schneidbereich 25 weg gerichtete Teil der Durchlassbereiche 22d, 23d eignet sich in besonderem Maße für eine Wiederaufbereitung, also Reinigung des Instruments nach erfolgtem Eingriff oder auch für das nachträgliche Aufbringen einer Beschichtung der Teilungsflächen 22e, 22e', 23e, 23e' mit einer beispielsweise verschleißresistenten Keramik, da durch die Ausgestaltung der Durchlassbereiche 22d, 23d eine verbesserte Zugänglichkeit gewährleistet ist.

Auch die in Fig. 6 gezeigte Elektrodenanordnung ist im Wesentlichen in Fig. 2 beschrieben. Das mit einer konkaven Krümmung 23b ausgebildete Elektrodenteil 23 weist an Endbereichen ein sägezahnartiges Profil 27, 27' auf. Die Zähne, können beispielsweise so angeordnet sein, dass sie während einem Zusammenführen der Branchen immer weiter in das Gewebe 40 greifen und dieses in einer Zugrichtung Z mitnehmen. Damit wird die Spannung im Gewebe 40 deutlich erhöht. Es ist allerdings darauf zu achten, dass durch das Profil 27, 27' eine Verletzung des Gewebes 40 vermieden wird, so dass die Zähne vorzugsweise als Noppen ausgebildet sind.

Vorzugsweise sind die Noppen derart angeordnet, dass das Gewebe 40 bei einem leichten Öffnen der Branchen durch das Profil 27, 27' in seiner gespannten Position gehalten wird. Das Profil 27, 27' fungiert demgemäß als eine Anordnung von Widerhaken.

Vorteilhafterweise kann ein zwischen den Elektrodenflächen angeordneter Isolationsabschnitt als ein einen Spanneffekt der Spannbereiche unterstützendes Oberflächenprofil ausgebildet sein. Damit wird auf einfachste Weise sowohl die Vermeidung des Kurzschlusses zwischen den Elektrodenteilen vermieden, als auch das Spannen des Gewebes verstärkt.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 10: Elektrochirurgisches Instrument
- 11: Distales Ende
- 12: Distales Ende
- 13: Proximales Ende
- 14: Proximales Ende
- 15: Branche
- 16: Branche
- 17: Achse
- 18: Griffteil
- 19: Griffteil
- 20: Stromanschlusselement, Stromzuführungseinrichtung
- 21: Stromanschlusselement, Stromzuführungseinrichtung
- 22: Elektrodenteil
- 22a: Koagulationsfläche
- 22b: Konvexe Krümmung
- 22c: Spannbereich
- 22d: Durchlassbereich
- 22e, 22e': Teilungsfläche
- 23: Elektrodenteil
- 23a: Koagulationsfläche
- 23b: Konkave Krümmung
- 23c: Spannbereich
- 23d: Durchlassbereich
- 23e, 23e': Teilungsfläche
- 24: Führungsspalt
- 25: Schneidbereich
- 27, 27': Profil
- 28: Isolationsabschnitt
- 28a, 28a': Teilabschnitt des Isolationsabschnitts
- 30: Schneidinstrument
- 31: Klinge
- 32: Fingerschalter
- 40: Gewebe, Gefäß
- Z: Zugrichtung

## Patentansprüche

1. Elektrochirurgisches Instrument mit
- zwei gelenkig miteinander verbundenen Branchen (15, 16), die entsprechend einem Schneid- oder Klemmwerkzeug betätigbar sind,
- einander gegenüberliegenden Elektrodenteilen (22, 23) mit Koagulationsflächen (22a, 23a) an distalen Enden (11, 12) der Branchen (15, 16) zum Fassen von einem Gefäß oder Gewebe (40) und zum Durchleiten eines Koagulationsstromes durch das Gefäß oder Gewebe (40) zu dessen Koagulation,
- Stromzuführungseinrichtungen (20, 21) zum Zuführen des Koagulationsstromes zu den Elektrodenteilen (22, 23) von einem HF-Generator,
wobei eine der Koagulationsflächen (22a) mindestens in einem ersten mittleren Abschnitt konvex, die ihr gegenüberliegende Koagulationsfläche (23a) mindesten in einem zweiten mittleren Abschnitt konkav gekrümmt ist, **dadurch gekennzeichnet, dass** ein Krümmungsradius der konkav gekrümmten Koagulationsfläche (23a) mindestens in dem zweiten mittleren Abschnitt größer ist als ein Krümmungsradius der konvex gekrümmten Koagulationsfläche (22a) in dem ersten mittleren Abschnitt und wobei die Krümmungen (22b, 23b) um Längsachsen der distalen Enden (11, 12) derart verlaufen, dass das zwischen den distalen Enden (11, 12) gehaltene und senkrecht zu den Längsachsen verlaufende Gefäß, oder Gewebe (40) mit zu dem ersten und zweiten mittleren Abschnitt hin steigender Pressung gehalten wird.

2. Elektrochirurgisches Instrument nach Anspruch 1,
**dadurch gekenntzeichnet**, dass
an mindestens einer der Koagulationsflächen im mittleren Abschnitt ein Isolationsabschnitt (28) ausgebildet ist, so dass ein direkter elektrischer Kontakt zwischen den Koagulationsflächen (22a, 23a) vermeidbar ist.

3. Elektrochirurgisches Instrument nach Anspruch 1 oder 2, insbesondere nach Anspruch 2,
**dadurch gekennzeichnet, dass**
der Isolationsabschnitt (28) derart an der mindestens einen Koagulationsfläche ausgebildet ist, dass er über diese hervorsteht.

4. Elektrochirurgisches Instrument nach einem der vorhergehenden Ansprüche, insbesondere nach Anspruch 2 oder 3,
**dadurch gekennzeichnet, dass**
der Isolationsabschnitt (28) aus mehreren Teilabschnitten ausgebildet ist.

5. Elektrochirurgisches Instrument nach einem der vorhergehenden Ansprüche, insbesondere nach Anspruch 2,
**dadurch gekennzeichnet, dass**
der Isolationsabschnitt (28) derart an der mindestens einen Koagulationsfläche ausgebildet ist, dass er sich durchgängig entlang einer Scheitellinie der Koagulationsfläche (22a, 23a) erstreckt und im Wesentlichen bündig mit dieser abschließt.

6. Elektrochirurgisches Instrument nach einem der vorhergehenden Ansprüche, insbesondere nach einem der Ansprüche 2 bis 5,
**dadurch gekennzeichnet, dass**
der Isolationsabschnitt (28) aus Keramik oder Diamant ausgebildet ist.

7. Elektrochirurgisches Instrument nach einem der vorhergehenden Ansprüche,
**dadurch gekennzeichnet, dass**
mindestens ein Elektrodenteil (22, 23) mindestens einen Durchlassbereich (22d, 23d) für ein Schneidinstrument (30) zum Durchführen eines Schneidvorgangs aufweist, so dass mindestens ein Abschnitt des Durchlassbereichs (22d, 23d) als Führungsspalt (24) für das Schneidinstrument (30) vorgesehen und dieses an dem eingeklemmten Gewebe (40) ansetzbar ist.

8. Elektrochirurgisches Instrument nach einem der vorhergehenden Ansprüche, insbesondere nach Anspruch 7,
**dadurch gekennzeichnet, dass**
der Durchlassbereich (22d, 23d) die jeweiligen Elektrodenteile (22, 23) in mindestens zwei Bereiche teilt, so dass die Elektrodenteile (22, 23) jeweils sich gegenüberliegende, parallel zueinander angeordnete Teilungsflächen (22e, 22e', 23e, 23e') aufweisen.

9. Elektrochirurgisches Instrument nach einem der vorhergehenden Ansprüche, insbesondere nach Anspruch 7,
**dadurch gekennzeichnet, dass**
der Durchlassbereich (22d, 23d) die jeweiligen Elektrodenteile (22, 23) in mindestens zwei Bereiche teilt, so dass die Elektrodenteile (22, 23) jeweils sich gegenüberliegende, sich in Richtung der Koagulationsflächen (22a, 23a) verjüngend zueinander angeordnete Teilungsflächen (22e, 22e', 23e, 23e') aufweisen.

10. Elektrochirurgisches Instrument nach einem der vorhergehenden Ansprüche, insbesondere nach einem der Ansprüche 7 bis 9,
**dadurch gekennzeichnet, dass**
Durchlassbereiche (22d, 23d) an den gegenüberliegenden Elektrodenteilen (22, 23) vorgesehen sind, die bei zusammengeführten Branchen (15, 16) im Wesentlichen fluchtend aneinander grenzen.

11. Elektrochirurgisches Instrument nach einem der vorhergehenden Ansprüche, insbesondere nach einem der Ansprüche 7 bis 10,
**gekennzeichnet durch** ein
Schneidinstrument (30), welches mit dem elektrochirurgischen Instrument (10) verbunden ausgebildet ist.

12. Elektrochirurgisches Instrument nach einem der vorhergehenden Ansprüche, insbesondere nach einem der Ansprüche 7 bis 11,
**gekennzeichnet durch** ein
Schneidinstrument (30), welches mechanisch und/oder elektrisch betätigbar ist.

13. Elektrochirurgisches Instrument nach einem der vorhergehenden Ansprüche, insbesondere nach einem der Ansprüche 7 bis 12,
**gekennzeichnet durch** ein
Schneidinstrument (30), welches zum Schneiden mittels eines HF-Stromes ausgebildet und mit einer Steuerungseinheit verbindbar ist, so dass der Schneidstrom in Abhängigkeit von Operationsphasen zuführbar ist.

14. Elektrochirurgisches Instrument nach einem der vorhergehenden Ansprüche,
**dadurch gekennzeichnet, dass**
an dem einen Elektrodenteil und/oder an dem gegenüberliegenden Elektrodenteil ein einen durch die Krümmungen (22b. 23b) bewirkten Spanneffekt an dem Gewebe (40) unterstützendes Oberflächenprofil (27, 27') ausgebildet ist.

15. Elektrochirurgisches Instrument nach einem der vorhergehenden Ansprüche, insbesondere nach Anspruch 14,
**dadurch gekennzeichnet, dass**
das den Spanneffekt unterstützende Oberflächenprofil (27, 27') als Sägezahnprofil ausgebildet ist.

16. Elektrochirurgisches Instrument nach einem der vorhergehenden Ansprüche, insbesondere nach Anspruch 14 oder 15,
**dadurch gekennzeichnet, dass**
der oder jeder Isolationsabschnitt (28) als das den Spanneffekt unterstützendes Oberflächenprofil (27, 27') ausgebildet ist.

17. Elektrochirurgisches Instrument nach einem der vorhergehenden Ansprüche,
**dadurch gekennzeichnet, dass**
das Instrument als laparoskopisches Instrument ausgebildet ist.

## Claims

1. Electrosurgical instrument, having
- two branches (15, 16) which are interconnected in an articulated manner and which can be operated analogously to a cutting or clamping tool,
- oppositely disposed electrode sections (22, 23) with coagulation surfaces (22a, 23a) at distal ends (11, 12) of the branches (15, 16) for gripping a vessel or tissue (40) and for conducting a coagulation current through the vessel or tissue (40) for its coagulation,
- current feed devices (20, 21) for feeding the coagulation current to the electrode sections (22, 23) from an HF generator,
wherein
one of the coagulation surfaces (22a) is convexly curved, at least in a first centre section, and the coagulation surface (23a) opposite to it is concavely curved, at least in a second centre section,
**characterized in that**
a curvature radius of the concavely curved coagulation surface (23a), at least in the second centre section, is larger than a curvature radius of the convexly curved coagulation surface (22a) in the first centre section, and wherein the curvatures (22b, 23b) extend around longitudinal axes of the distal ends (11, 12) in such a way that the vessel or tissue (40), which is held between the distal ends (11, 12) and extends perpendicularly to the longitudinal axes, is held with increasing pressure towards the first and second centre sections.

2. Electrosurgical instrument according to Claim 1,
**characterized in that**
an insulating section (28) is formed on at least one of the coagulation surfaces in the centre section so that a direct electrical contact between the coagulation surfaces (22a, 23a) can be avoided.

3. Electrosurgical instrument according to Claim 1 or 2, especially according to Claim 2,
**characterized in that**
the insulating section (28) is formed on the at least one coagulation surface in such a way that it projects beyond this.

4. Electrosurgical instrument according to one of the preceding claims, especially according to Claim 2 of 3,
**characterized in that**
the insulating section (28) is formed from a plurality of sub-sections.

5. Electrosurgical instrument according to one of the preceding claims, especially according to Claim 2,
**characterized in that**
the insulating section (28) is formed on the at least one coagulation surface in such a way that it extends continuously along a vertex line of the coagulation surface (22a, 23a) and terminates essentially flush with this.

6. Electrosurgical instrument according to one of the preceding claims, especially according to one of Claims 2 to 5,
**characterized in that**
the insulating section (28) is formed from ceramic or diamond.

7. Electrosurgical instrument according to one of the preceding claims,
**characterized in that**
at least one electrode section (22, 23) has at least one opening region (22d, 23d) for a cutting instrument (30) for carrying out a cutting process so that at least one section of the opening region (22d, 23d) is provided as a guide gap (24) for the cutting instrument (30) and this cutting instrument can be positioned on the clamped tissue (40).

8. Electrosurgical instrument according to one of the preceding claims, especially according to Claim 7,
**characterized in that**
the opening region (22d, 23d) splits the respective electrode sections (22, 23) into at least two regions so that the electrode sections (22, 23) have in each case oppositely disposed, separated surfaces (22e, 22e', 23e, 23e') which are arranged parallel to each other.

9. Electrosurgical instrument according to one of the preceding claims, especially according to Claim 7,
**characterized in that**
the opening region (22d, 23d) splits the respective electrode sections (22, 23) into at least two regions so that the electrode sections (22, 23) have in each case oppositely disposed, separated surfaces (22e, 22e', 23e, 23e') which are arranged in a manner in which they taper towards each other in the direction of the coagulation surfaces (22a, 23a).

10. Electrosurgical instrument according to one of the preceding claims, especially according to one of Claims 7 to 9,
**characterized in that**
provision is made on the oppositely disposed electrode sections (22, 23) for opening regions (22d, 23d) which, when the branches (15,16) are brought together, abut essentially in alignment.

11. Electrosurgical instrument according to one of the preceding claims, especially according to one of Claims 7 to 10,
**characterized by** a
cutting instrument (30), which is designed for connecting to the electrosurgical instrument (10).

12. Electrosurgical instrument according to one of the preceding claims, especially according to one of Claims 7 to 11,
**characterized by** a
cutting instrument (30), which can be operated mechanically and/or electrically.

13. Electrosurgical instrument according to one of the preceding claims, especially according to one of Claims 7 to 12,
**characterized by** a
cutting instrument (30), which is designed for cutting by means of an HF current and which can be connected to a control unit so that the cutting current can be supplied in dependence upon phases of the operation.

14. Electrosurgical instrument according to one of the preceding claims,
**characterized in that**
a surface profile (27, 27') is formed on the one electrode section and/or on the oppositely disposed electrode section and assists a clamping effect on the tissue (40) which is brought about as a result of the curvatures (22b, 23b).

15. Electrosurgical instrument according to one of the preceding claims, especially according to Claim 14,
**characterized in that**
the surface profile (27, 27'), which assists the clamping effect, is formed as a sawtooth profile.

16. Electrosurgical instrument according to one of the preceding claims, especially according to Claim 14 or 15,
**characterized in that**
the insulating section (28), or each insulating section, is formed as the surface profile (27, 27') which assists the clamping effect.

17. Electrosurgical instrument according to one of the preceding claims,
**characterized in that**
the instrument is designed as a laparoscopic instrument.

## Revendications

1. Instrument d'électrochirurgie comprenant
- deux branches (15, 16) reliées l'une à l'autre de façon articulée, qui peuvent être actionnées au moyen d'un outil de coupe ou de serrage,
- des parties d'électrode (22, 23) se faisant face avec des surfaces de coagulation (22a, 23a) sur des extrémités distales (11, 12) des branches (15, 16) pour la saisie d'un vaisseau ou d'un tissu (40) et pour le passage d'un flux de coagulation dans le vaisseau ou le tissu (40) pour sa coagulation,
- des dispositifs d'arrivée de courant (20, 21) pour l'arrivée du flux de coagulation aux parties d'électrode (22, 23) provenant d'un générateur H.F.,
une des surfaces de coagulation (22a) étant incurvée de façon convexe au moins dans une première partie centrale, la surface de coagulation (23a) qui fait face étant incurvée de façon concave au moins dans une seconde partie centrale, **caractérisé en ce qu'**un rayon de courbure de la surface de coagulation (23a) à courbure concave est plus grand, au moins dans la seconde partie centrale, qu'un rayon de courbure de la surface de coagulation (22a) incurvée de façon convexe dans la première partie centrale et les courbures (22b, 23b) étant agencées autour d'axes longitudinaux des extrémités (11, 12) distales, de telle sorte que le vaisseau ou le tissu (40) maintenu entre les extrémités distales (11, 12) et agencé perpendiculairement aux axes longitudinaux est maintenu avec une compression augmentant en direction de la première et de la seconde partie centrale.

2. Instrument d'électrochirurgie selon la revendication 1,
**caractérisé en ce**
**qu'**une partie d'isolation (28) est conçue sur au moins l'une des surfaces de coagulation dans la partie centrale, de telle sorte qu'un contact électrique direct entre les surfaces de coagulation (22a, 23a) peut être évité.

3. Instrument d'électrochirurgie selon la revendication 1 ou 2, en particulier selon la revendication 2,
**caractérisé en ce que**
la partie d'isolation (28) est formée directement sur la au moins une surface de coagulation, de telle sorte qu'elle dépasse de celle-ci.

4. Instrument d'électrochirurgie selon l'une des revendications précédentes, en particulier selon la revendication 2 ou 3,
**caractérisé en ce que**
la partie d'isolation (28) est conçue à base de plusieurs tronçons partiels.

5. Instrument d'électrochirurgie selon l'une des revendications précédentes, en particulier selon la revendication 2,
**caractérisé en ce que**
la partie d'isolation (28) est conçue sur la au moins une surface de coagulation, de telle sorte qu'elle s'étend de façon continue le long d'une ligne de crête de la surface de coagulation (22a, 23a) et se termine sensiblement au niveau de celle-ci.

6. Instrument d'électrochirurgie selon l'une des revendications précédentes, en particulier selon l'une des revendications 2 à 5,
**caractérisé en ce que**
la partie d'isolation (28) est conçue à base de céramique ou de diamant.

7. Instrument d'électrochirurgie selon l'une des revendications précédentes,
**caractérisé en ce**
**qu'**au moins une partie d'électrode (22, 23) présente au moins une zone de passage (22d, 23d) pour un instrument de coupe (30) pour l'exécution d'une opération de coupe, de sorte qu'au moins une partie de la zone de passage (22d, 23d) est prévue comme fente de guidage (24) pour l'instrument de coupe (30) et que cette partie peut être placée sur le tissu (40) coincé à l'intérieur.

8. Instrument d'électrochirurgie selon l'une des revendications précédentes, en particulier selon la revendication 7,
**caractérisé en ce que**
la zone de passage (22d, 23d) divise les parties d'électrode (22, 23) respectives en au moins deux zones, de sorte que les parties d'électrode (22, 23) présentent des surfaces de division (22e, 22e', 23e, 23e') se faisant face respectivement et disposées parallèlement entre elles.

9. Instrument d'électrochirurgie selon l'une des revendications précédentes, en particulier selon la revendication 7,
**caractérisé en ce que**
la zone de passage (22d, 23d) divise les parties d'électrode (22, 23) respectives en au moins deux zones, de sorte que les parties d'électrode (22, 23) présentent des surfaces de division (22e, 22e', 23e, 23e') se faisant face respectivement et disposées l'une par rapport à l'autre en se rétrécissant en direction des surfaces de coagulation (22a, 23a).

10. Instrument d'électrochirurgie selon l'une des revendications précédentes, en particulier selon l'une des revendications 7 à 9,
**caractérisé en ce que**
des zones de passage (22d, 23d) sont prévues sur les parties d'électrode (22, 23) se faisant face, lesquelles sont sensiblement contiguës en alignement dans le cas de branches (15, 16) guidées ensemble.

11. Instrument d'électrochirurgie selon l'une des revendications précédentes, en particulier selon l'une des revendications 7 à 10,
**caractérisé par** un instrument de coupe (30), lequel est conçu relié à l'instrument (10) d'électrochirurgie.

12. Instrument d'électrochirurgie selon l'une des revendications précédentes, en particulier selon l'une des revendications 7 à 11,
**caractérisé par** un instrument de coupe (30), lequel peut être actionné de façon mécanique et/ou électrique.

13. Instrument d'électrochirurgie selon l'une des revendications précédentes, en particulier selon l'une des revendications 7 à 12,
**caractérisé par** un instrument de coupe (30), lequel est conçu pour couper au moyen d'un courant de haute fréquence et peut être relié à une unité de commande, de sorte que le flux de coupe peut être amené en fonction de phases d'opération.

14. Instrument d'électrochirurgie selon l'une des revendications précédentes,
**caractérisé en ce**
**qu'**un profil de surface (27, 27') favorisant un effet de serrage, provoqué par les courbures (22b, 23b), sur le tissu (40) est conçu sur l'une des parties d'électrode et/ou sur la partie d'électrode opposée.

15. Instrument d'électrochirurgie selon l'une des revendications précédentes, en particulier selon la revendication 14,
**caractérisé en ce que**
le profil de surface (27, 27') favorisant l'effet de serrage est conçu en forme de profil en dents de scie.

16. Instrument d'électrochirurgie selon l'une des revendications précédentes, en particulier selon la revendication 14 ou 15,
**caractérisé en ce que**
la ou chaque partie d'isolation (28) est conçue comme profil de surface (27, 27') favorisant l'effet de serrage.

17. Instrument d'électrochirurgie selon l'une des revendications précédentes,
**caractérisé en ce que**
l'instrument est conçu comme un instrument laparoscopique.
